# EUROPÄISCHE PATENTANMELDUNG

(11) **EP 2 090 287 A2**
(43) Veröffentlichungstag der Anmeldung: **19.08.2009**
(21) Anmeldenummer: 08020785.5
(22) Anmeldetag: 29.11.2008
(51) Int. Cl.: A61K 8/35, A61Q 19/06, A61Q 19/00, A61K 8/34, A61Q 19/08

(54) **Kosmetische oder dermatologische Zubereitungen mit einem Gehalt an 3-(4-Hydroxy-3-methoxyphenyl)-1-(4-hydroxyphenyl)-propan-1-on und einem oder mehreren Polyolen**

(30) Priorität: 18.02.2008 DE 102008009750
(71) Anmelder: Beiersdorf Aktiengesellschaft, 20245 Hamburg (DE)
(72) Erfinder: Kolbe, Ludger, 21255 Dohren (DE); Eckert, Julia, 22085 Hamburg (DE); Neufang, Gitta, 20149 Hamburg (DE); Wöhrmann, Michael, 22525 Hamburg (DE); Knaupmeier, Stefanie, 22391 Hamburg (DE); Peters, Nils, 22965 Todendorf (DE)
(74) Vertreter: Wilke, Jochen

(57) **Zusammenfassung**

Kosmetische oder dermatologische Zubereitungen mit einem Gehalt an 3-(4-Hydroxy-3-methoxyphenyl)-1-(4-hydroxyphenyl)-propan-1-on und einem oder mehreren Polyolen.

## Beschreibung

Die vorliegende Erfindung betrifft kosmetische oder dermatologische Zubereitungen mit einem Gehalt an 3-(4-Hydroxy-3-methoxyphenyl)-1-(4-hydroxyphenyl)-propan-1-on und einem oder mehreren Polyolen.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten. Dabei sollten die Zubereitungen insbesondere auch die Hautfeuchtigkeit verbessern.

Es hat sich überraschenderweise herausgestellt, daß eine kosmetische Zubereitung, dadurch gekennzeichnet, daß sie eine kosmetisch wirksame Menge an 3-(4-Hydroxy-3-methoxyphenyl)-1-(4-hydroxyphenyl)-propan-1-on und ein oder mehreren Polyole enthält, den Nachteilen des Standes der Technik abhilft.

Erfindungsgemäß wird eine vom pH-Wert unabhängige Löslichkeit von 3-(4-Hydroxy-3-methoxyphenyl)-1-(4-hydroxyphenyl)-propan-1-on erreicht.

Vorteilhafte Beispiele für Poylyole sind: Erythrit, Arabit, Adonit, Sorbit, Dulcit, 1,2-Butandiol, 1,3-Butandiol, 1,4-Butandiol, 2,3-Butandiol, 1,5-Pentandiol, 2,4-Pentandiol, 2-Methyl-1,3-Propandiol, 1,2,3-Propantriol, 2-Methyl-2,4-pentandiol, 1,6-Hexandiol, 2,3-Dimethyl-2,3-butandiol, 2,2-Diethyl-1,3-propandiol, 2-Ethyl-1,3-hexandiol, 2,2,4-Trimethyl-1,3-pentandiol, 2-Ethyl-2-butyl-1,3-propandiol, 1,2,4-Butantriol, 1,2,6-Hexantriol, 2,2-Dihydroxymethyl-1-butanol, 1,2-Octandiol,Tetramethylolmethan, dem α-Mono-methylether von Glycerin, dem α-Mono-n-butylether von Glycerin, 2-O-α-D-Glucopyranosyl-L-ascorbinsäure, 3-(2-Ethylhexyloxy)-1,2-propandiol, Ethylenglycolmonophenylether, 1,2-Hexandiol.

Bevorzugt sind Polyole, die 2 bis 3 Sauerstoffatome enthalten und mindestens 3 Kohlenstoffatome.

Das Verhältnis von 3-(4-Hydroxy-3-methoxyphenyl)-1-(4-hydroxyphenyl)-propan-1-on zu Polyol (bzw. der Gesamtmenge an Polyolen) sollte 1:2 bis 1:1000, insbesondere 1:5 bis 1:500 betragen.

Die Verwendung erfindungsgemäßer Zubereitungen mit einem kosmetisch wirksamen Gehalt an 3-(4-Hydroxy-3-methoxyphenyl)-1-(4-hydroxyphenyl)-propan-1-on - z. B. in Form der genannten Beispiele - führt in überraschender Weise zu einer erheblichen Verbesserung des Aussehens der Haut, insbesondere werden durch Anwenden erfindungsgemäßer Zubereitungen
● die Barriereeigenschaften der Haut erhalten oder wiederhergestellt,
● besser der Hautaustrocknung entgegengewirkt sowie
● die Haut besser vor Umwelteinflüssen geschützt.

Durch die Verwendung erfindungsgemäßer Zubereitungen wird ferner die Hautfeuchtigkeit und die Tiefenfeuchtigkeit der Haut gesteigert, wobei diese hautbefeuchtende Pflege-Wirkung und der Schutz vor Feuchtigkeitsverlust über mehrere Stunden bzw. sogar über den ganzen Tag oder noch länger anhält.

Erfindungsgemäß ist daher auch die Verwendung kosmetischer Zubereitungen, die eine kosmetisch wirksame Menge an 3-(4-Hydroxy-3-methoxyphenyl)-1-(4-hydroxyphenyl)-propan-1-on enthalten, zur Steigerung der Hautfeuchtigkeit bzw. zur Befeuchtung der Haut.

Es war ferner überraschend, dass durch Anwenden erfindungsgemäßer Zubereitungen die Elastizität der Haut, der Haare und/oder der Nägel und daher Cellulite bzw. das Erscheinungsbild der sog. "Orangenhaut" verbessert wird.

Bevorzugt enthalten kosmetische oder dermatologische Zubereitungen gemäß der Erfindung 0,001 bis 30 Gew.-%, bevorzugt 0,01 bis 15 Gew.-%, besonders bevorzugt 0,1 bis 5 Gew.-% an 3-(4-Hydroxy-3-methoxyphenyl)-1-(4-hydroxyphenyl)-propan-1-on, bezogen auf das Gesamtgewicht der Zubereitung.

Herstellungsverfahren für das 3-(4-Hydroxy-3-methoxyphenyl)-1-(4-hydroxyphenyl)-propan-1-on 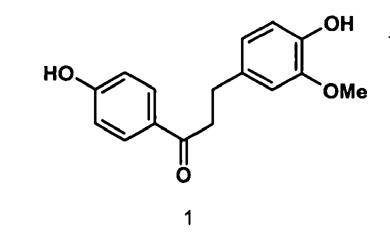 können vorteilhaft aus den folgenden Schritten bestehen oder diese umfassen:
a) Umsetzung von p-Hydroxyacetophenon mit Vanillin zu 3-(4-Hydroxy-3-methoxyphenyl)-1-(4-hydroxyphenyl)-2-propen-1-on und
b) Hydrierung des in Schritt a) gewonnenen 3-(4-Hydroxy-3-methoxyphenyl)-1-(4-hydroxyphenyl)-2-propen-1-on zu 3-(4-Hydroxy-3-methoxyphenyl)-1-(4-hydroxyphenyl)-propan-1-on.

In einer bevorzugten Ausgestaltung des erfindungsgemäßen Herstellverfahrens für die Verbindung der Formel 1 wird die Umsetzung von p-Hydroxyacetophenon mit Vanillin in Schritt a) so durchgeführt, daß p-Hydroxyacetophenon und Vanillin einer Mischung aus Kaliumhydroxid in Diethylenglykoldiethylether bei erhöhter Temperatur zugesetzt wird. Die Temperatur liegt üblicherweise im Bereich von 50 bis 200 °C, bevorzugt im Bereich von 80°C bis 140°C, weiter bevorzugt im Bereich von 100 bis 120°C.

Anschließend wird die entstandene Verbindung bevorzugt zu 3-(4-Hydroxy-3-methoxyphenyl)-1-(4-hydroxyphenyl)-2-propen-1-on hydrolysiert. Bevorzugt wird der pH-Wert im Bereich von 6 bis 7 eingestellt.

In einer bevorzugten Ausgestaltung des erfindungsgemäßen Herstellverfahrens für die Verbindung der Formel 1 wird die Hydrierung des in Schritt (a) gewonnenen 3-(4-Hydroxy-3-methoxyphenyl)-1-(4-hydroxyphenyl)-2-propen-1-on zu 3-(4-Hydroxy-3-methoxyphenyl)-1-(4-hydroxyphenyl)-propan-1-on so durchgeführt, daß das entstandene 3-(4-Hydroxy-3-methoxyphenyl)-1-(4-hydroxyphenyl)-2-propen-1-on in Tetrahydrofuran gelöst und mit einer geeigneten Menge Pd auf Aktivkohle (Pd-Gehalt: 5 Gew.-%, Wassergehalt ca. 50 Gew.-%, jeweils bezogen auf die Gesamtmasse des Katalysators) versetzt wird. Vorzugsweise wird die Reaktion bei Normaldruck und weiter vorzugsweise bei Raumtemperatur (ca. 20°C) vorgenommen.

### Synthesebeispiel für 3-(4-Hydroxy-3-methoxyphenyl)-1-(4-hydroxyphenyl)-1-propanon

### (a) Umsetzung von p-Hydroxyacetophenon mit Vanillin:

Es werden 20 g Kaliumhydroxid in 100 g Diethylenglykoldiethylether vorgelegt, auf 120°C unter Rühren erhitzt und mit einer Mischung von 14 g p-Hydroxyacetophenon und 15 g Vanillin innerhalb von 1 h versetzt. Nach beendeter Dosierung rührt man noch 20 min nach, hydrolysiert und stellt auf pH 6-7 ein. Nach Phasentrennung entfernt man das Lösungsmittel und erhält 25 g 3-(4-Hydroxy-3-methoxyphenyl)-1-(4-hydroxyphenyl)-2-propen-1-on, Ausbeute: 93% der Theorie.

### (b) Hydrierung des in Schritt (a) gewonnenen Reaktionsprodukts:

10 g 3-(4-Hydroxy-3-methoxyphenyl)-1-(4-hydroxyphenyl)-2-propen-1-on werden in 100 g Tetrahydrofuran gelöst, mit 0,2 g Pd auf Aktivkohle (Pd-Gehalt: 5 Gew.-%, Wassergehalt ca. 50 Gew.-%, jeweils bezogen auf die Gesamtmasse des Katalysators) versetzt und bei Normaldruck und Raumtemperatur (ca. 20°C) hydriert. Man erhält nach Entfernung des Katalysators und des Lösungsmittels 9 g an 3-(4-Hydroxy-3-methoxyphenyl)-1-(4-hydroxyphenyl)-propan-1-on. Ausbeute: 89% der Theorie

Spektroskopische Daten von 3-(4-Hydroxy-3-methoxyphenyl)-1-(4-hydroxyphenyl)-propan-1-on (Formel 1):
¹³C-NMR (CDCl₃; 75,5 MHz): δ (ppm) = 197,42(s), 161,85(s), 147,24(s), 144,44(s), 132,03(s), 130,37(d), 130,37(d), 128,18(s), 120,27(d), 115,11(d), 115,07(d), 115,07(d), 112,53(d), 55,42(q), 39,30(t), 29,42(t);
MS: m/z (%) = M⁺-lon 272(82), 151(24), 137(77), 121(100), 93(19), 65(22).

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an 3-(4-Hydroxy-3-methoxyphenyl)-1-(4-hydroxyphenyl)-propan-1-on.

Die kosmetischen oder dermatologischen Zubereitungen enthalten ferner bevorzugt 0,001 Gew.-% bis 30 Gew.-%, bevorzugt 0,05 Gew.-% bis 10 Gew.-%, insbesondere 0,1 - 6,0 Gew.-%, bezogen auf das Gesamtgewicht der Zubereitungen, an einem oder mehreren Polyolen.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, 3-(4-Hydroxy-3-methoxyphenyl)-1-(4-hydroxyphenyl)-propan-1-on in verkapselter Form darzureichen, z.B. in Kollagenmatrices und anderen üblichen Verkapselungsmaterialien, z.B. als Celluloseverkapselungen, in Gelatine, in Wachskapseln, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, 3-(4-Hydroxy-3-methoxyphenyl)-1-(4-hydroxyphenyl)-propan-1-on und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Vorteilhaft werden die Antioxidantien gewählt aus der Gruppe bestehend α-Hydroxysäuren (z.B. Citronensäure, Milchsäure, Apfelsäure), EDTA, Ubichinon und Ubichinol und deren Derivate (insbesondere Ubichinon Q10), Vitamin C und Derivate (z.B. Ascorbylpalmitat, Na-Ascorbylphosphat), Tocopherole und Derivate (z.B. Vitamin-E-acetat), Rutinsäure und deren Derivate insbesondere α-Glycosylrutin, Butylhydroxytoluol.

Auch ist die Verwendung der erfindungsgemäßen kosmetischen Zubereitungen zur Prophylaxe und Behandlung der Symptome von Altershaut, zur Verhinderung und Verminderung der Entstehung und Ausbreitung von Fältchen und Falten sowie zur Behandlung und Pflege gealterter Haut erfindungsgemäß. Geeignete Wirkstoffe für diesen Einsatzzweck sind: Ubichinon, Retinol und Derivate, Isoflavonoide (insbes. Genistein, Daidzein), Creatin, Phytoöstrogene, Niacinamid, Polyphenole oder eine andere, gegen Falten wirksame Substanz auf die Gesichtshaut auftragen und verteilen.

Weiterhin ist die Verwendung der erfindungsgemäßen kosmetischen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojobaöl), Ceramide, insbesondere Ceramid I, III und VI, Cholesterin, Phytosterole, Fettsäuren mit einer Kettenlänge von C16-26, insbesondere Linolsäure, Carnitin und seine Derivate, Harnstoff, Glycerin, Pseudoceramide; Elektrolyte wie Natriumchlorid und Taurin.

Außerdem ist die Verwendung der erfindungsgemäßen kugelförmigen kosmetischen Zubereitungen zur Prophylaxe und Behandlung der Symptome der fehlpigmentierten Haut vorteilhaft. Bevorzugte Wirkstoffe für diesen Einsatzzweck sind: Tyrosinaseinhibitoren, Hydrochinonderivate, Dioic Acid, Arbutin, Ellagsäure, Liponsäure und ihre Derivate, Vitamin C und Derivate sowie Kojisäure.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Sonnenblumenöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane insbesondere Cylcometicon, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, lsononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Bevorzugte O/W Emulgatoren sind Glycerylstearatcitrat, Polyglyceryl-3-methylglucosedistearat, Cetearylglucoside, PEG-40-Steartat, PEG-100-Stearat, Stearinsäure, Ceteareth-20, Steareth-20, Steareth-21 und Glycerylstearat

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| Whitening Lotion mit Lichtschutz, enthaltend 3-(4-Hydroxy-3-methoxyphenyl)-1-(4-hydroxyphenyl)-1-propanon | **Gew.-%** |
|---|---|
| Cetyl Alcohol | 3 |
| Glyceryl Stearate SE | 1.5 |
| Butyl Methoxydibenzoylmethane | 3 |
| Hydrogenated Coco-Glycerides | 1 |
| Ethylhexyl Salicylate | 4 |
| Ethylhexyl Methoxycinnamate | 7 |
| Butylene Glykol | 4 |
| EDTA | 0.2 |
| Tocopheryl Acetate | 0.5 |
| Benzophenone-3 | 3 |
| Phenoxyethanol | 0.4 |
| Dimethicone | 2 |
| Nylon 12 | 2 |
| Distarch Phosphate | 3 |
| Xanthan Gum | 0.4 |
| Glycerin | 7 |
| Phenylbenimidazole Sulfonic Acid | 2 |
| Alcohol Denat. | 4 |
| Ethylhexyglycerin | 0.2 |
| Phenoxyethanol | 0,5 |
| Ethylparaben | 0.1 |
| Methylparaben | 0.2 |
| Propylparaben | 0.1 |
| 3-(4-Hydroxy-3-methoxyphenyl)-1-(4-hydroxyphenyl)-1-propanon | 0.2 |
| Wasser | ad 100 |

| W/O-Creme mit 3-(4-Hydroxy-3-methoxyphenyl)-1-(4-hydroxyphenyl)-1-propanon | **Gew.-%** |
|---|---|
| Polyglyceryl-3-Diisostearat | 5,0 |
| Polyglyceryl-2-Dipolyhydroxystearat | 2,5 |
| Cetearylalkohol | 2 |
| Cetylalkohol | 2 |
| C₁₂₋₁₅ Alkylbenzoat | 8 |
| Caprylsäure/Caprinsäuretriglyceride | 6 |
| Octyldodecanol | 5 |
| Octamethyltetrasiloxan (Cyclomethicon) | 2 |
| Milchsäure | 0,5 |
| Ubichinon (Coenzym Q10) | 0,1 |
| Butylmethoxydibenzoylmethan | 2 |
| Ethylhexyltriazon | 1 |
| Titandioxid | 1 |
| Ethylhexylmethoxycinnamat | 5 |
| 3-(4-Hydroxy-3-methoxyphenyl)-1-(4-hydroxyphenyl)-1-propanon | 0.1 |
| Natriumpolyacrylat | 0,15 |
| Retinylpalmitat | 0,05 |
| p-Hydroxybenzoesäurealkyleste (Paraben) | 0,1 |
| Glycerin | 7 |
| Füllstoffe (EDTA, BHT) | 0,6 |
| Propylene Glykol | 3 |
| Wasser | Ad 100 |

| After Shave Gel mit 3-(4-Hydroxy-3-methoxyphenyl)-1-(4-hydroxyphenyl)-1-propanon | **Gew.-%** |
|---|---|
| Triceteareth-4-Phsophate | 1,0 |
| Cyclometicone | 2,0 |
| Octyldodecanol | 2,0 |
| Dicaprylylcarbonat | 3,0 |
| Methylpalmitat | 1,0 |
| 3-(4-Hydroxy-3-methoxyphenyl)-1-(4-hydroxyphenyl)-1-propanon | 0.15 |
| Aloe-Extrakt | 0,1 |
| Tocopherylacetat | 0,5 |
| Panthenol | 0,2 |
| Ethanol | 5,0 |
| Phenoxyethanol | 0,5 |
| p-Hydroxybenzoesäurealkylester (Paraben) | 0,4 |
| Carbopol Ultrez 10 | 0,1 |
| Distärkephosphat | 1,0 |
| Methylpropandiol | 3,0 |
| Glycerin | 4,0 |
| Parfüm | q.s. |
| Wasser | ad 100 |

| Sensitiv Balm mit 3-(4-Hydroxy-3-methoxyphenyl)-1-(4-hydroxyphenyl)-1-propanon | **Gew.-%** |
|---|---|
| Glycerylsterat | 2,5 |
| PEG-40-Stearat | 1 |
| Cetearylalkohol | 2 |
| Hydrierte Kokosfettglyceride (Hydrogenated Coco Glycerides) | 1 |
| Myristylmyristate | 2 |
| C₁₂₋₁₅ Alkylbenzoat | 4 |
| Caprylsäure/Caprinsäuretriglyceride | 2 |
| Octyldodecanol | 1 |
| Dicaprylylcarbonat | 3 |
| Jojobaöl | 1 |
| Ethylhexylcyanodiphenylacrylat (Octocrylen) | 5 |
| Bis-Ethylhexyloxyphenol-methoxyphenyltriazin | 2 |
| 3-(4-Hydroxy-3-methoxyphenyl)-1-(4-hydroxyphenyl)-1-propanon | 0.5 |
| Alpha-Glucosylrutin | 0,1 |
| Citronensäure | 0,3 |
| Methylparaben | 0,3 |
| 1,2-Octandiol | 0,5 |
| C10-30 Alkyl/Acrylates Crosspolymer | 0,2 |
| Tapiocapartikel | 1 |
| Glycerin | 10 |
| Additive (Distärkephosphat, EDTA, BHT) | 0.5 |
| Parfüm | q.s. |
| Wasser | ad 100 |

## Patentansprüche

1. Kosmetische oder dermatologische Zubereitungen mit einem Gehalt an 3-(4-Hydroxy-3-methoxyphenyl)-1-(4-hydroxyphenyl)-propan-1-on und einem oder mehreren Polyolen.

2. Zubereitungen nach Anspruch 1, **dadurch gekennzeichnet, daß** das oder die Polyole gewählt werden aus der Gruppe Erythrit, Arabit, Adonit, Sorbit, Dulcit, 1,2-Butandiol, 1,3-Butandiol, 1,4-Butandiol, 2,3-Butandiol, 1,5-Pentandiol, 2,4-Pentandiol, 2-Methyl-1,3-Propandiol, 1,2,3-Propantriol, 2-Methyl-2,4-pentandiol, 1,2-Hexandiol, 1,6-Hexandiol, 2,3-Dimethyl-2,3-butandiol, 2,2-Diethyl-1,3-propandiol, 2-Ethyl-1,3-hexandiol, 2,2,4-Trimethyl-1,3-pentandiol, 2-Ethyl-2-butyl-1,3-propandiol, 1,2,4-Butantriol, 1,2,6-Hexantriol, 2,2-Dihydroxymethyl-1-butanol, 1,2-Octandiol,Tetramethylolmethan, dem α-Mono-methylether von Glycerin, dem α-Mono-n-butylether von Glycerin, Ethylenglycolmonophenylether, 2-O-α-D-Glucopyranosyl-L-ascorbinsäure, 3-(2-Ethylhexyloxy)-1,2-propandiol,.

3. Zubereitungen nach Anspruch 1, **dadurch gekennzeichnet, daß** das oder die Polyole gewählt werden aus der Gruppe 1,3-Butandiol , 1,2,3-Propantriol ,1,5-Pentandiol, 2-Methyl-1,3-Propandiol, 2-Methyl-2,4-pentandiol, 1,6-Hexandiol, 1,2-Octandiol, 3-(2-Ethylhexyloxy)-1,2-propandiol, 1,2-Hexandiol.

4. Zubereitungen nach einem der vorstehenden Ansprüche, **dadurch gekennzeichnet, daß** sie 0,001 Gew.-% bis 10 Gew.-%, bevorzugt 0,01 Gew.-% bis 1,0 Gew.-%, insbesondere 0,05 - 0,5 Gew.-%, bezogen auf das Gesamtgewicht der Zubereitungen, an 3-(4-Hydroxy-3-methoxyphenyl)-1-(4-hydroxyphenyl)-propan-1-on enthalten.

5. Zubereitungen nach einem der vorstehenden Ansprüche, **dadurch gekennzeichnet, daß** sie 0,001 Gew.-% bis 30 Gew.-%, bevorzugt 0,05 Gew.-% bis 10 Gew.-%, insbesondere 0,1 - 6,0 Gew.-%, bezogen auf das Gesamtgewicht der Zubereitungen, an einem oder mehreren Polyolen.

6. Zubereitungen nach einem der vorstehenden Ansprüche, **dadurch gekennzeichnet, daß** das Verhältnis von 3-(4-Hydroxy-3-methoxyphenyl)-1-(4-hydroxyphenyl)-propan-1-on zu Polyol (bzw. der Gesamtmenge an Polyolen) aus dem Bereich 1:2 bis 1:1000, insbesondere 1:5 bis 1:500 gewählt wird.
